# DEMANDE DE BREVET EUROPEEN

(11) **EP 1 759 688 A1**
(43) Date de publication de la demande: **07.03.2007**
(21) Numéro de dépôt: 06118683.9
(22) Date de dépôt: 09.08.2006
(51) Int. Cl.: A61K 8/368, A61Q 19/00, A61Q 19/10

(54) **Procédé cosmétique de soin des peaux grasses et kit associé**

(30) Priorité: 05.09.2005 FR 0509061
(71) Demandeur: L'Oreal, 75008 Paris (FR)
(72) Inventeur: Compain, Delphine, 75012, Paris (FR)
(74) Mandataire: Leonard, Armelle

(57) **Abrégé**

La présente invention se rapporte à un procédé cosmétique de soin de la peau, destiné à atténuer les imperfections cutanées des peaux grasses, comprenant l'application successive sur la peau de trois compositions, à savoir : une composition de nettoyage, une composition de peeling, et une composition de soin dont chacune a une constitution donnée.

Elle se rapporte également à un kit comprenant trois types de conditionnement dont chacun renferme l'une des compositions précitées.

## Description

La présente invention se rapporte à un procédé cosmétique de soin de la peau, destiné à atténuer les imperfections cutanées des peaux grasses, comprenant l'application successive sur la peau de trois compositions, à savoir : une composition de nettoyage, une composition de peeling, et une composition de soin dont chacune a une constitution donnée.

Elle se rapporte également à un kit comprenant trois types de conditionnement dont chacun renferme l'une des compositions précitées.

La brillance de la peau est un problème affectant plus particulièrement les adolescents mais qui peut aussi se manifester à l'âge adulte sous l'effet notamment d'une hyperproduction d'androgènes. Une peau brillante est généralement une peau hyper-séborrhéique caractérisée par une sécrétion et une excrétion exagérées de sébum conduisant généralement à un taux de sébum supérieur à 200 µg/cm² mesuré au niveau du front.

Le sébum est le produit naturel de la glande sébacée qui constitue une annexe de l'unité pilosébacée. Il s'agit essentiellement d'un mélange plus ou moins complexe de lipides. Classiquement, la glande sébacée produit du squalène, des triglycérides, des cires aliphatiques des cires de cholestérol et, éventuellement du cholestérol libre (Stewart,M.E., Semin Dermatol 11, 100-105 (1992)). L'action des lipases bactériennes convertit une part variable des triglycérides formés en acides gras libres.

Le sébocyte constitue la cellule compétente de la glande sébacée. La production de sébum est associée à un programme de différenciation terminale de cette cellule. Durant cette différenciation, l'activité métabolique du sébocyte est essentiellement axée sur la biosynthèse des lipides *(la lipogenèse)* et plus précisément sur la néosynthèse d'acide gras.

Si le sébum constitue normalement un hydratant naturel de l'épiderme, la surproduction de sébum peut entraîner des désagréments esthétiques (peau luisante, moins bonne tenue du maquillage, formation de comédons), voire constituer un terrain propice au développement anarchique de la flore bactérienne saprophyte telle que P. *acnes* et provoquer des lésions acnéiques.

Pour lutter contre l'hyperséborrhée, il a donc été proposé dans l'art antérieur divers composés qui, par application topique sur la peau, sont susceptibles de diminuer la lipogenèse au niveau des sébocytes et limiter, par voie de conséquence, la production de sébum.

Parmi ces composés, on peut citer notamment un extrait de grains de café (FR-2 848 447) et l'acide ascorbique et ses dérivés (EP-0 771 557).

Il est par ailleurs courant d'introduire, dans les produits cosmétiques destinés au soin des peaux grasses, des poudres d'origine naturelle ou synthétique destinées à absorber le sébum, parmi lesquelles on peut citer notamment les charges telles que le talc, l'amidon, la silice et les poudres de polyamide (Nylon). La Demanderesse a par ailleurs mis en évidence (EP-1 493 433) que des particules poreuses de polyamide ayant un diamètre moyen, en volume, inférieur ou égal à 10 µm pouvaient être chargées d'actifs destinés notamment au soin des peaux grasses et permettre ainsi de véhiculer ces actifs dans le follicule pilo-sébacé, sur leur site d'action.

Enfin, les imperfections cutanées des peaux grasses sont classiquement traitées par introduction, dans les compositions destinées au soin de ces peaux, de composés kératolytiques ayant pour effet de lutter contre l'hyperkératinisation responsable de l'obstruction des canaux pilo-sébacés, et/ou de composés anti-bactériens destinés à lutter contre la prolifération bactérienne au sein de ces canaux, et/ou de composés anti-inflammatoires destinés à limiter l'inflammation consécutive à cette prolifération bactérienne.

Les produits cosmétiques de soin des peaux grasses à imperfections disponibles sur le marché présentent toutefois l'inconvénient d'être soit insuffisamment efficaces, soit trop agressifs sur une peau déjà lésée et irritée. La peau est alors desséchée et sensible. Outre l'inconfort qui en résulte, le dessèchement de la peau peut entraîner en réaction une surproduction de sébum qui accentue la brillance de la peau.

Il subsiste donc le besoin de disposer d'une méthode permettant de lutter efficacement contre les imperfections cutanées des peaux grasses, de façon immédiate et à long terme, sans dessécher la peau, c'est-à-dire sans inconfort

Or, la Demanderesse a découvert qu'il était possible de satisfaire ce besoin en suivant un protocole mettant en oeuvre trois compositions appliquées successivement. Ce protocole permet d'atteindre un juste équilibre entre efficacité et tolérance du traitement.

La présente invention a ainsi pour objet un procédé cosmétique destiné à atténuer les imperfections cutanées d'une peau grasse, comprenant l'application successive sur une zone de ladite peau :
- d'une composition de nettoyage contenant au moins 0,1% en poids d'au moins un agent desquamant, qui est appliquée sur la peau, puis éliminée par rinçage,
- d'une composition de peeling renfermant au moins 5% en poids d'au moins un agent desquamant, et
- d'une composition de soin renfermant au moins un composé choisi parmi : les agents desquamants, les agents anti-séborrhéiques, les agents matifiants, les agents anti-bactériens, les agents apaisants et leurs mélanges.

Par "peau grasse", on entend une peau luisante, qui présente en particulier un taux de sébum supérieur à 200 µg/cm² mesuré au niveau du front.

De préférence, la zone de peau sur laquelle le procédé ci-dessus est mis en oeuvre est une zone de peau du visage, de préférence tout le visage excepté le contour des yeux.

Par 'imperfections des peaux grasses' selon la présente invention, on entend notamment la brillance de la peau et la formation des comédons.

La première étape du procédé selon l'invention, ci-après désignée par étape de nettoyage, consiste à appliquer sur la peau une composition renfermant au moins 0,1% en poids d'au moins un agent desquamant. Cette première étape a pour but d'assurer une bonne adhérence sur la peau de la composition de peeling faisant l'objet de la deuxième étape.

Par « agent desquamant», on entend dans le cadre de cette description un composé d'origine naturelle ou synthétique, qui a pour effet d'éliminer de façon chimique (et non mécanique) les cellules mortes de la couche cornée, par exemple en stimulant la dégradation de la cornéodesmosine ou en stimulant le renouvellement épidermique (notamment par stimulation de la prolifération épidermique).

L'agent desquamant peut être notamment choisi parmi : les α-hydroxyacides tels que l'acide citrique, lactique, glycolique, malique, tartrique ou mandélique ; les β-hydroxyacides tels que l'acide salicylique et ses dérivés, en particulier l'acide n-octanoyl-5-salicylique ; l'urée et ses dérivés tels que l'hydroxyéthyl urée ; les composés aminosulfoniques et en particulier l'acide (N-2 hydroxyéthylpiperazine-N-2-éthane) sulfonique (HEPES) ; les dérivés de sucre et en particulier le 0-octanoyl-6-D-maltose et le miel ; l'acide 8-hexadécène-1,16-dicarboxylique ; les dérivés de l'acide 2-oxothiazolidine-4-carboxylique (procystéine) ; un extrait de Saphora japonica ; le resvératrol ; l'acide cinnamique ; et l'acide jasmonique et ses dérivés tels que l'acide (1R,2R) 3-hydroxy-2-pentyl-cyclopentane-acétique.

Comme agents desquamants préférés, on pourra utiliser les α-hydroxyacides tels que l'acide citrique, lactique, glycolique, malique, tartrique ou mandélique ; les β-hydroxyacides tels que l'acide salicylique et ses dérivés, en particulier l'acide n-octanoyl-5-salicylique ; l'urée et ses dérivés tels que l'hydroxyéthyl urée ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; un extrait de Saphora japonica.

L'acide salicylique et ses dérivés, notamment l'acide n-octanoyl-5-salicylique est particulièrement préféré pour une utilisation dans la présente invention.

L'agent desquamant peut représenter de 0,1 à 10%, et de préférence de 0,1 à 5%, et plus préférentiellement de 0,1 à 2%, mieux, de 0,5 à 0,6% du poids total de la composition de nettoyage mise en oeuvre dans la première étape du procédé selon l'invention. Cette composition a de préférence un pH allant de 4 à 9, de préférence de 5 à 7.

La composition utilisée dans la première étape du procédé selon l'invention peut se présenter sous forme de gel ou d'émulsion, par exemple. Elle peut comprendre différents adjuvants et renferme avantageusement au moins un tensioactif moussant, qui peut être un tensioactif non ionique, amphotère, anionique ou cationique, ou un mélange de tels tensioactifs. Ce tensioactif moussant a généralement un HLB supérieur à 20.

De préférence, la composition utilisée dans la première étape du procédé selon l'invention se présente sous forme d'un gel, en particulier d'un gel aqueux et de préférence sous forme d'un gel aqueux moussant.

Comme tensioactifs moussants non ioniques, la composition peut contenir par exemple, un ou plusieurs tensioactifs non ioniques choisis parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Les tensioactifs moussants amphotères et zwitterioniques peuvent être choisis par exemple parmi les dérivés de bétaïnes dont les amidopropylbétaïnes, les amphoacétates, les hydroxylsultaines et leurs mélanges.

Les tensioactifs anioniques peuvent être choisis par exemple parmi les savons (sels alcalins d'acides gras), les dérivés d'aminoacides comme les acylaminoacide tels que les glycinates, les amidoéther carboxylates, les alkyl polyaminocarboxylates, les alkyl éthers sulfates tels que les Sodium Laureth sulfates, les alkyl sulfonates, les iséthionates, les alkyl méthyltaurate, les alkyl sulfosuccinates, les alkyl sulfoacétates, les alkylphosphates (monoalkylphosphates ou dialkylphosphates), les acides alkyl glycol carboxyliques tel que l'acide lauryl glycol carboxylique commercialisé notamment par la société SANYO sous la dénomination commerciale BEAULIGHT SHAA (Acid Form), leurs sels, et leurs mélanges.

La composition de nettoyage selon l'invention peut en outre contenir un ou plusieurs émulsionnants n'ayant pas de propriétés moussantes mais ayant en revanche la capacité de faciliter la dispersion de deux phases insolubles l'une dans l'autre. Ces émulsionnants ont généralement un HLB allant de 3 à 18.

Elle peut par ailleurs renfermer des huiles hydrocarbonées, fluorées, siliconées ou végétales ou des esters non émulsifiants d'alcools gras et/ou d'acides gras ; ainsi que des actifs lipophiles et/ou hydrophiles autres que l'agent desquamant mentionné plus haut, des polyols tels que la glycérine ou le propylène glycol, et des conservateurs, sans que cette liste soit limitative.

Selon un mode particulier, la composition de nettoyage utilisée dans la première étape du procédé selon l'invention ne contient pas d'huiles.

Cette composition peut être préparée selon des procédés bien connus de l'homme du métier, notamment par chauffage de chacune des phases huileuse et aqueuse puis introduction de l'une de ces phases dans l'autre, dans le cas des émulsions, ou par la méthode dite d'inversion de phase en température.

L'application de cette composition peut se faire par massage du bout des doigts, ou à l'aide d'une lingette, d'une éponge ou de tout autre support imprégné passé sur le visage, par exemple. La composition est ensuite laissée en contact avec la peau pendant une durée allant de préférence de cinq secondes à une minute avant d'être éliminée par rinçage. Pour ce faire, la peau sera généralement rincée à l'eau.

La seconde étape du procédé selon l'invention, ou étape de peeling, comprend l'application sur la peau d'une composition renfermant au moins 5% en poids d'au moins un agent desquamant.

Cet agent desquamant peut être notamment choisi parmi ceux listés précédemment.

Comme agents desquamants préférés, on pourra utiliser les α-hydroxyacides tels que l'acide citrique, lactique, glycolique, malique, tartrique ou mandélique ; les β-hydroxyacides tels que l'acide salicylique et ses dérivés, en particulier l'acide n-octanoyl-5-salicylique ; l'urée et ses dérivés tels que l'hydroxyéthyl urée ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; un extrait de Saphora japonica.

L'acide lactique, l'acide glycolique, l'acide salicylique et leurs mélanges sont préférés pour une utilisation dans la seconde étape de ce procédé. Un mélange d'acide salicylique et d'acide glycolique est plus préférentiellement utilisé. Il a en effet été découvert que ce mélange permettait d'obtenir un effet desquamant à la fois sur les couches profondes et sur les couches superficielles de l'épiderme, en raison des différentes tailles et longueurs de chaîne des molécules qui le constituent.

La quantité d'agent desquamant peut représenter de 5 à 15%, et de préférence de 5 à 10%, du poids de la composition de peeling mise en oeuvre dans la seconde étape du procédé selon l'invention. On peut ainsi utiliser de 1 à 3%, et de préférence 2%, d'acide salicylique et de 3 à 7%, et de préférence 5%, d'acide glycolique, par rapport au poids total de la composition.

Cette composition a de préférence un pH compris entre 2,5 et 6, avantageusement entre 3,5 et 4,5.

Elle peut être appliquée sur la peau sous forme de masque (formé in situ à partir d'un hydrogel, ou préformé sur un support non-tissé) ou de patch (ayant des propriétés adhésives) ou être imprégnée sur une lingette. Ces formes d'exécution ont l'avantage de permettre un conditionnement de la composition sous forme de mono-doses évitant l'application sur la peau d'une quantité excessive d'agent desquamant susceptible de générer des effets indésirables. Elles permettent en outre de respecter un temps de pause minimal, qui dépendra de la nature et de la concentration de l'agent desquamant, assurant l'obtention du résultat voulu.

En variante, on peut utiliser la composition de peeling sous forme de dispersions du type lotion, gel, ou émulsions, en particulier sous forme de lotion renfermant le ou les agents desquamant(s) dans un milieu aqueux ou hydro-alcoolique, l'alcool étant choisi parmi l'éthanol et l'isopropanol et de préférence constitué d'éthanol. Cette forme galénique permet une bonne pénétration du ou des actifs, convient aux peaux acnéiques, s'étale bien et sèche rapidement. Dans le cas où on utilise une lotion hydro-alcoolique, la quantité d'alcool dans la composition de peeling est avantageusement comprise entre 60 et 90%, mieux, voisine de 80%, par rapport au poids total de la composition de peeling.

Selon un mode préféré, la composition de peeling est sous la forme de dispersions du type lotion, gel, ou émulsions, en particulier sous forme d'une lotion.

La composition de peeling peut contenir divers adjuvants tels que des agents émulsionnants, des polymères amphiphiles ioniques, des gélifiants et des neutralisants. L'ajout d'un gélifiant est en particulier avantageux pour permettre un étalement facilité de la composition de peeling sur la peau.

Elle est généralement laissée en contact avec la peau pendant une durée allant d'une minute à cinq minutes.

Cette étape de peeling peut ou non être suivie d'une étape dite de neutralisation consistant à appliquer sur la peau une composition ramenant le pH de la peau à une valeur proche de 6. Cette neutralisation peut par exemple être obtenue par pulvérisation d'eau thermale sur la peau.

La troisième étape du procédé selon l'invention comprend l'application sur la peau d'une composition de soin renfermant au moins un composé choisi parmi :
- les agents desquamants ;
- les agents séborégulateurs ;
- les agents matifiants ;
- les agents anti-bactériens ;
- les agents apaisants ; et
- leurs mélanges.

De préférence, la composition selon l'invention comprend un mélange de deux, trois, quatre ou cinq des composés ci-dessus, étant entendu qu'un même composé peut exercer plusieurs des fonctions citées précédemment. Par exemple, elle peut comprendre un mélange d'un composé desquamant, d'un composé matifiant et d'un composé apaisant et séborégulateur.

Les agents desquamants utilisables dans la troisième composition selon l'invention peuvent être choisis parmi ceux cités précédemment. On préfère utiliser l'acide salicylique.

La quantité d'agent desquamant peut représenter de 0,05 à 5%, et de préférence de 0,1 à 1% en poids, par rapport au poids total de la troisième composition.

Dans le cadre de cette description, on entend par "agent séborégulateur" un composé réduisant la production de sébum par les sébocytes.

Des exemples d'agents séborégulateurs utilisables dans la présente invention sont : les rétinoïdes tels que le rétinol ; l'acide ascorbique et ses dérivés tels que le glucoside d'ascorbyle et l'ascorbyl phosphate de magnésium ; les sels organiques et inorganiques de zinc et en particulier le zinc PCA et le gluconate de zinc ; les extraits de laminaires et en particulier de Laminaria saccharina tels que ceux vendus par la société SECMA sous la dénomination commerciale Phlorogine ; et un extrait de Spiraea ulmaria commercialisé notamment par la société SILAB sous la dénomination commerciale Sebonormine®.

Par "glucoside d'ascorbyle", on entend le produit de condensation du glucose, sous forme D, c'est-à-dire sous forme de glucopyrannose α ou β ou de furannose α ou β, ou sous forme L, avec l'acide ascorbique, de préférence sous forme L. Le 2-O-α-D-glucopyranoside d'acide L-ascorbique est préféré pour une utilisation dans la présente invention. Il est notamment disponible auprès de la société HAYASHIBARA.

Un composé séborégulateur particulièrement préféré pour une utilisation dans la présente invention est un extrait de Laminaria saccharina.

La quantité d'agent séborégulateur utilisable dans la troisième composition selon l'invention peut varier de 0,1 à 5% en poids, de préférence de 1 à 5% en poids, et mieux, de 2 à 4% en poids, par rapport au poids total de la composition.

Par « agent matifiant », on entend un composé réduisant la brillance de la peau, généralement par absorption et/ou adsorption du sébum ou encore en modifiant la trajet des rayons lumineux sur ou dans la peau. L'effet matifiant du composé peut être évalué dans un solvant approprié en mesurant au moyen d'un gonioréflectomètre le rapport R entre la réflexion spéculaire et la réflexion diffuse. Une valeur de R inférieure ou égale à 2 traduit généralement un effet matifiant.

Les agents matifiants agissant par absorption et/ou adsorption du sébum sont encore désignés par « pompes à sébum ».

Généralement, ce type de composé se présente sous la forme d'une poudre de particules ayant une prise de sébum. De manière avantageuse, la prise de sébum de ces composés est supérieure ou égale à 1 ml/g, notamment supérieure ou égale à 2 ml/g, et en particulier supérieure ou égale à 3 ml/g. La prise de sébum correspond à la quantité de sébum absorbée et/ou adsorbée sur la surface disponible des particules. Elle est mesurée selon la méthode de Wet Point.

Les particules de composé absorbant le sébum peuvent présenter une densité tassée inférieure ou égale à 2 g/cm³, notamment inférieure ou égale à 1 g/cm³, et en particulier inférieure ou égale à 0,5 g/cm³.

Cette densité est appréciée selon le protocole suivant :
On verse m = 40 g de poudre dans une éprouvette graduée ; puis on place l'éprouvette sur l'appareil STAV 2003 de chez STAMPF VOLUMETER ; l'éprouvette est ensuite soumise à 1500 tassements ; puis on mesure directement sur l'éprouvette le volume final Vf de poudre tassée. La densité tassée est déterminée par le rapport m/Vf, en l'occurrence 40/Vf (m étant exprimé en g et Vf en cm³).

Selon un mode de réalisation particulier, les particules de composé absorbant et/ou adsorbant le sébum peuvent présenter une surface spécifique BET supérieure ou égale à 300 m²/g, notamment supérieure ou égale à 500 m²/g, et en particulier supérieure ou égale à 600 m²/g, et notamment inférieure ou égale à 1500 m²/g.

La « surface spécifique BET » est déterminée selon la méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale (donc micropores compris) des particules considérées.

Les particules de composé absorbant et/ou adsorbant le sébum peuvent être d'origine minérale ou organique.

Plus précisément, ce composé peut être choisi parmi la silice, les silicates mixtes, les poudres de polyamide (nylon®), les poudres de polymères acryliques, notamment de polyméthacrylate de méthyle, de polyméthacrylate de méthyle/diméthacrylate d'éthylène glycol, de polyméthacrylate d'allyle/diméthacrylate d'éthylène glycol et de copolymère diméthacrylate d'éthylène glycol/méthacrylate de lauryle, et leurs mélanges.

Les particules de ce composé peuvent, le cas échéant, être traitées en surface par au moins un agent de traitement hydrophobe, notamment non fluoré, tel que des silicones ou des acides aminés.

A titre représentatif et non limitatif des composés absorbant et/ou adsorbant le sébum selon l'invention, on peut tout particulièrement citer :
- les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33" , "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
- les poudres de silicates mixtes amorphes, notamment d'aluminium et de magnésium, comme par exemple celle commercialisée sous la dénomination « NEUSILIN UFL2 » par la société Sumitomo.
- les poudres de polyamides (nylon®), comme par exemple "I'ORGASOL® 4000" commercialisé par la société ATOCHEM, et
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING.
   Selon une variante particulière, le composé absorbant et/ou adsorbant le sébum est d'origine minérale.

Selon un mode de réalisation tout particulièrement avantageux, les particules de composé absorbant et/ou adsorbant le sébum sont des particules de silicate et notamment de silicate mixte, et en particulier d'aluminium et de magnésium.

Dans le cadre de cette invention, on préfère utiliser les argiles telles que le kaolin.

Des exemples d'agents matifiants réduisant la brillance de la peau par effet optique, encore désignés par « charges soft-focus », sont les dispersions aqueuses de charge colloïdale inorganique telle que la silice ou un composite silice-alumine.

La quantité d'agent matifiant utilisable dans la troisième composition selon l'invention peut varier de 0,1 à 5% en poids, de préférence de 1 à 5% en poids, et mieux, de 2 à 4% en poids, par rapport au poids total de la composition.

Par « agent anti-bactérien », on entend des composés ayant un effet bactériostatique sur les germes de l'acné et en particulier sur *P. acnes.* Ces agents anti-microbiens peuvent notamment être choisis parmi le 2,4,4'-trichloro-2'-hydroxy diphényl éther (ou triclosan), l'acide phytique, l'acide N-acétyl-L-cystéine, l'acide lipoïque, l'acide azélaïque et ses sels, l'acide arachidonique, le résorcinol, l'octopirox ou piroctone olamine, l'octoxyglycérine, l'octanoylglycine, le caprylyl glycol, l'acide 10-hydroxy-2-décanoïque, les sels de cuivre tels que le pidolate de cuivre, l'acide salicylique, l'iodopropynyl butylcarbamate, le farnesol, les phytosphingosines et leurs mélanges.

L'agent anti-bactérien peut représenter de 0,01 à 5%, de préférence de 0,1 à 2%, du poids total de la troisième composition.

Enfin, par « agent apaisant », on entend un composé réduisant les sensations d'échauffement, de prurit, de picotements ou de tiraillements, qui sont généralement choisis parmi les antagonistes de substance P, les antagonistes de CGRP et les antagonistes de bradykinine ou encore parmi les composés anti-inflammatoires.

Des exemples de telles substances sont : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, le bisabolol, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et Nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, les extraits de Centella asiatica, un extrait de Rosa gallica, un extrait de Mentha piperita (Calmiskin de SILAB), le piperonal, un extrait de clou de girofle, un extrait d'Epilobium Angustifolium, l'aloe vera, un extrait de Bacopa monieri, les phytostérols, et la niacinamide. Par "extraits", on entend en particulier les extraits hydroglycoliques des plantes mentionnées ci-dessus.

Les agents apaisants préférés pour une utilisation dans la présente invention sont les extraits de Rosa gallica et de Mentha piperita.

L'agent apaisant peut représenter de 0,01 à 5%, de préférence de 0,1 à 2%, du poids total de la troisième composition.

L'un ou plusieurs des composés ci-dessus, et en particulier les agents séborégulateurs, peuvent être véhiculés dans des systèmes de ciblage folliculaire tels que ceux décrits dans la demande EP-1 493 433 dont le contenu est incorporé ici par référence. Il peut s'agir en particulier de particules poreuses de polyamide ayant un diamètre moyen en volume inférieur ou égal à 10µm.

En outre, le pH de la troisième composition selon l'invention est de préférence compris entre 5 et 7 et va de préférence de 5,5 à 6,5, pour permettre à la peau de retrouver son pH normal.

Cette composition comprend généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner la consommatrice d'utiliser cette composition.

Elle peut se présenter sous toutes les formes galéniques classiquement utilisées pour une application topique et notamment sous forme de dispersions du type lotion ou gel, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition mise en oeuvre dans la troisième étape du procédé selon l'invention se présente sous la forme d'une émulsion huile-dans-eau (H/E). Les huiles présentes dans cette émulsion peuvent être des huiles de silicone, volatiles ou non, des huiles hydrocarbonées, des huiles végétales. Cette émulsion peut en outre comprendre des corps gras non huileux, tels que du beurre de karité, des gommes de silicone, des esters d'acides gras et d'alcools gras, des acides gras et des alcools gras.

Cette composition peut en outre contenir divers adjuvants couramment utilisés dans le domaine cosmétique, tels que des émulsionnants dont les esters d'acide gras et de glycéryle, les esters d'acides gras et de sucre, les esters d'acide gras et de sorbitane, les esters d'acide gras et polyéthylèneglycol, les alcools gras éthoxylés et les alkylpolyglycosides ; des conservateurs et/ou des co-conservateurs tels que le caprylyl glycol ; des séquestrants tels que les sels d'EDTA ; des colorants ; des parfums ; des ajusteurs de pH tels que des neutralisants et/ou des tampons ; de l'éthanol ; et des épaississants et gélifiants, en particulier les homo- et copolymères d'acrylamide, les homo- et copolymères acryliques, les homo- et copolymères d'acide acrylamidométhylpropane sulfonique (AMPS) et la gomme de xanthane.

Bien entendu, l'homme du métier veillera à choisir ce ou ces éventuels composés additionnels et/ou leur quantité de manière telle que les propriétés avantageuses de la composition de soin utilisée selon l'invention ne soient pas, ou substantiellement pas, altérées par l'adjonction envisagée.

Les compositions selon l'invention ont de préférence un pH acide ou faiblement acide allant de 2.5 à 7, avec par exemple :
- un pH faiblement acide allant de 5 à 7 pour les 1^{ère} et 3^{è} étapes ;
- un pH acide allant de 2.5 à 6, de préférence de 3.5 à 4.5 pour la 2^{è} étape.

Les compositions utilisées dans le procédé selon l'invention peuvent être disposées dans un conditionnement commun ou "kit".

La présente invention a également pour objet un kit comprenant :
- un premier conditionnement renfermant une composition de nettoyage qui contient au moins 0,1% en poids d'au moins un agent desquamant,
- au moins un deuxième conditionnement renfermant une composition qui contient au moins 5% en poids d'au moins un agent desquamant,
- un troisième conditionnement renfermant une composition qui contient au moins un composé choisi parmi : les agents desquamants, les agents anti-séborrhéiques, les agents matifiants, les agents anti-bactériens, les agents apaisants et leurs mélanges.

Les termes "premier", "deuxième" et "troisième" conditionnements utilisés ci-dessus n'ont pas pour effet de différencier les conditionnements sur le plan de leur apparence, mais sur le plan de la composition qu'ils contiennent. Certains ou tous peuvent donc se présenter sous la même forme physique.

Toutefois, selon une forme d'exécution préférée de l'invention, le premier conditionnement peut se présenter sous forme de tube, ou éventuellement de flacon pompe, de préférence ayant une contenance allant de 30 à 50 ml.

Le deuxième conditionnement peut être une bouillotte, c'est-à-dire un flacon monodose, éventuellement accompagnée d'un doseur gradué permettant l'administration d'une quantité adéquate (efficace et bien tolérée) de composition de peeling sur la peau.

Le troisième conditionnement peut se présenter sous forme de flacon-pompe, de tube ou de pot, par exemple, qui peut notamment avoir une contenance de 30 à 50 ml.

Le kit décrit ci-dessus peut également contenir une notice renfermant des indications sur son mode d'utilisation, pour la mise en oeuvre du procédé décrit précédemment.

Un mode d'utilisation préférentiel, encore désigné par "routine", comprend la mise en oeuvre du procédé décrit précédemment un jour sur deux, de préférence le soir, pendant une durée pouvant aller de 3 à 6 semaines et de préférence égale à 4 semaines.

L'invention sera maintenant illustrée par les exemples non limitatifs suivants. Dans ces exemples, les quantités sont indiquées en pourcentage pondéral.

### EXEMPLES

### Exemple 1 : Kit pour peaux grasses

Un kit peut être été fabriqué, qui comprend un tube renfermant la composition A de nettoyage (première composition selon l'invention), la composition B de peeling (deuxième composition selon l'invention) et la composition C de soin (troisième composition selon l'invention) décrites ci-dessous.

Les ingrédients de ces compositions sont mentionnés en noms INCl ou en noms chimiques, selon le cas. Ces compositions peuvent être préparées de façon classique pour l'homme du métier. Elles sont destinées à être appliquées successivement sur la peau.

### Composition A de nettoyage

| **GEL MOUSSANT** | |
|---|---|
| **PHASE I** | |
| Eau | Qsp. |
| Glycérine | 5 % |
| Conservateurs | 0.4 % |
| Acide Salicylique | 0.55 % |

| **PHASE II** | |
|---|---|
| PEG-120 methyl glucose dioleate | 2 % |

| **PHASE III** | |
|---|---|
| Sodium laureth sulfate | 15 % |

| **PHASE IV** | |
|---|---|
| Decyl glucoside | 8 % |
| PEG-200 Hydrogenated glyceryl palmate (and) PEG-7 Glyceryl Cocoate | 10 % |
| Triethanolamine | 0.32 % |

Ce gel peut être appliqué le soir avant le coucher sur le visage mouillé, en évitant le contour des yeux et des lèvres, par massage du bout des doigts, suivi d'un rinçage à l'eau et d'un séchage à l'aide d'une serviette propre.

### Composition B de peeling

| **LOTION** | |
|---|---|
| **PHASE I** | |
| Alcool | 80 % |
| Hydroxypropylcellulose | 0.85 % |
| Acide Salicylique | 2 % |

| **PHASE II** | |
|---|---|
| PPG-12/SMDI Copolymer | 1 % |

| **PHASE III** | |
|---|---|
| Acide Glycolique | 5 % |
| Acrylates/octylacrylamide copolymer | 0.5 % |
| Glycérine | 0.5 % |

| **PHASE IV** | |
|---|---|
| Sodium hydroxide | 0.11 % |
| Eau | Qsp. |

Cette lotion peut être prélevée à l'aide d'une pipette graduée, à raison de 2,5 ml, dans une ***bouillotte**,* puis transférée sur un coton qui est ensuite appliqué sur la peau.

### Composition C de Soin

| | |
|---|---|
| | |

| **PHASE I** | |
|---|---|
| Eau | Qsp. |
| Glycérine | 7 % |
| Conservateurs | 0.45 % |
| Acide salicylique | 0.6 % |
| Kaolin | 3 % |

| **PHASE II** | |
|---|---|
| Glyceryl stearate SE | 1.5% |
| Arachidyl alcohol (and) behenyl alcohol (and) arachidyl glucoside | 1.5% |
| Cyclohexasiloxane | 10 % |

| **PHASE III** | |
|---|---|
| AMPS | 0.6 % |
| Xanthan Gum | 0.2 % |

| **PHASE IV** | |
|---|---|
| Extrait de Laminaria saccharina (Phlorogine de SECMA) | 0.5 % |

| **PHASE V** | |
|---|---|
| Alcool | 5 % |

| **PHASE VI** | |
|---|---|
| Sodium Hydroxide | 0.17 % |

Cette composition peut être conditionnée dans un tube, prélevée à la main et appliquée du bout des doigts sur l'ensemble du visage et du front.

### Exemple 2 : Test in vivo

### a) Protocole

La méthode selon l'invention a été testée sous contrôle dermatologique sur un panel de 43 femmes âgées de 18 à 45 ans ayant la peau mixte ou grasse et à tendance acnéique, présentant au minimum 5 lésions inflammatoires et/ou 10 rétentionnelles, avec un score de Sébutape (produit vendu par CUDERM et utilisé selon les indications du fournisseur) au niveau du front supérieur à 2.

Les sujets ont suivi pendant un mois le protocole suivant, à raison de trois fois par semaine :
1) Nettoyer le soir le visage avec la Composition A de l'Exemple 1 puis rincer abondamment à l'eau et sécher en tapotant à l'aide d'une serviette
2) Prélever 2,5ml de la Composition B de l'Exemple 1 à l'aide d'une pipette fournie. Déposer le produit sur un coton. Appliquer sur l'ensemble du visage et du cou en évitant le contour des yeux, des lèvres et des narines. Laisser poser 5 minutes.
3) Appliquer ensuite par dessus la Composition C de l'Exemple 1. Eviter le contour des yeux.

### b) Résultats

Les résultats de ce test montrent une bonne tolérance des compositions testées, ainsi qu'une bonne cosméticité.

Ainsi, 97% des femmes jugent que le gel nettoyant est agréable et 78% qu'il se rince facilement ; plus de 85% des femmes estiment que la lotion de peeling s'étale bien, pénètre facilement et sèche vite et 100% jugent que leur peau est au moins aussi bien qu'avec leur produit habituel ; et 94% des femmes considèrent que le soin hydratant est agréable. En outre, plus de 88% des felles jugent que ces produits conviennent bien à leur type de peau.

En outre, après 4 semaines de traitement à l'aide du kit ci-dessus, 94% des femmes jugent que ce kit a asséché leurs imperfections ; 86% que leurs pores sont purifiés ; 86% que leur teint est plus clair ; et 78% qu'il n'a pas desséché leur peau.

Ces résultats montrent que le kit et le procédé selon l'invention permettent de lutter efficacement contre les imperfections cutanées des peaux grasses tout en étant bien toléré.

## Revendications

1. Procédé cosmétique destiné à atténuer les imperfections cutanées d'une peau grasse, comprenant l'application successive sur une zone de ladite peau :
- d'une composition de nettoyage contenant au moins 0,1% en poids d'au moins un agent desquamant, qui est appliquée sur la peau, puis éliminée par rinçage,
- d'une composition de peeling renfermant au moins 5% en poids d'au moins un agent desquamant, et
- d'une composition de soin renfermant au moins un composé choisi parmi : les agents desquamants, les agents anti-séborrhéiques, les agents matifiants, les agents anti-bactériens, les agents apaisants et leurs mélanges.

2. Procédé selon la revendication 1, **caractérisé en ce que** ladite zone de peau est une zone de peau du visage.

3. Procédé selon la revendication 1 ou 2, **caractérisé en ce que** l'agent desquamant est choisi parmi : les α-hydroxyacides tels que l'acide citrique, lactique, glycolique, malique, tartrique ou mandélique ; les β-hydroxyacides tels que l'acide salicylique ou l'acide n-octanoyl-5-salicylique ; l'urée et ses dérivés tels que l'hydroxyéthyl urée ; les composés aminosulfoniques et en particulier l'acide (N-2 hydroxyéthylpiperazine-N-2-éthane) sulfonique (HEPES) ; les dérivés de sucre et en particulier le O-octanoyl-6-D-maltose et le miel ; l'acide 8-hexadécène-1,16-dicarboxylique ; les dérivés de l'acide 2-oxothiazolidine-4-carboxylique (procystéine) ; un extrait de Sophora japonica ; le resvératrol ; l'acide cinnamique ; et l'acide jasmonique et ses dérivés tels que l'acide (1R,2R) 3-hydroxy-2-pentyl-cyclopentane-acétique.

4. Procédé selon la revendication 3, **caractérisé en ce que** l'agent desquamant compris dans la composition de nettoyage est l'acide salicylique.

5. Procédé selon l'une quelconque des revendications 1 à 4, **caractérisé en ce que** l'agent desquamant représente de 0,1 à 2% du poids total de la composition de nettoyage.

6. Procédé selon l'une quelconque des revendications 1 à 5, **caractérisé en ce que** la composition de nettoyage a un pH allant de 4 à 9.

7. Procédé selon l'une quelconque des revendications 1 à 6, **caractérisé en ce que** l'agent desquamant compris dans la composition de peeling est choisi parmi : l'acide lactique, l'acide glycolique, l'acide salicylique et leurs mélanges.

8. Procédé selon l'une quelconque des revendications 1 à 7, **caractérisé en ce que** la quantité d'agent desquamant représente de 5 à 10%, du poids de la composition de peeling.

9. Procédé selon l'une quelconque des revendications 1 à 8, **caractérisé en ce que** la composition de peeling a un pH compris entre 3,5 et 4,5.

10. Procédé selon l'une quelconque des revendications 1 à 9, **caractérisé en ce que** l'agent séborégulateur est choisi parmi : les rétinoïdes tels que le rétinol ; l'acide ascorbique et ses dérivés tels que le glucoside d'ascorbyle et l'ascorbyl phosphate de magnésium ; les sels organiques et inorganiques de zinc et en particulier le zinc PCA et le gluconate de zinc ; les extraits de laminaires et en particulier de Laminaria saccharina ; et un extrait de Spiraea ulmaria

11. Procédé selon la revendication 10, **caractérisé en ce que** l'agent séborégulateur est un extrait de Laminaria saccharina.

12. Procédé selon l'une quelconque des revendications 1 à 11, **caractérisé en ce que** l'agent matifiant est choisi parmi : le kaolin et les dispersions aqueuses de charge colloïdale inorganique telle que la silice ou un composite silice-alumine.

13. Procédé selon l'une quelconque des revendications 1 à 12, **caractérisé en ce que** l'agent anti-bactérien est choisi parmi : le 2,4,4'-trichloro-2'-hydroxy diphényl éther (ou triclosan), l'acide phytique, l'acide N-acétyl-L-cystéine, l'acide lipoïque, l'acide azélaïque et ses sels, l'acide arachidonique, le résorcinol, l'octopirox ou piroctone olamine, l'octoxyglycérine, l'octanoylglycine, le caprylyl glycol, l'acide 10-hydroxy-2-décanoïque, les sels de cuivre tels que le pidolate de cuivre, l'acide salicylique, l'iodopropynyl butylcarbamate, le farnesol, les phytosphingosines et leurs mélanges.

14. Procédé selon l'une quelconque des revendications 1 à 13, **caractérisé en ce que** l'agent apaisant est choisi parmi : les triterpènes pentacycliques et les extraits de plantes (ex : Glycyrrhiza glabra) en contenant comme l'acide β-giycyrrhétinique et ses sels et/ou ses dérivés (l'acide glycyrrhétinique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, le bisabolol, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier muscat, de cassis, d'Echium, de poisson, des extraits de plancton, la capryloyl glycine, le Seppicalm VG (sodium palmitoylproline et Nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, les extraits de Centella asiatica, un extrait de Rosa gallica, un extrait de Mentha piperita (Calmiskin de SILAB), le piperonal, un extrait de clou de girofle, un extrait d'Epilobium Angustifolium, l'aloe vera, un extrait de Bacopa monieri, les phytostérols, et la niacinamide.

15. Kit comprenant :
- un premier conditionnement renfermant une composition de nettoyage qui contient au moins 0,1% en poids d'au moins un agent desquamant,
- au moins un deuxième conditionnement renfermant une composition qui contient au moins 5% en poids d'au moins un agent desquamant,
- un troisième conditionnement renfermant une composition qui contient au moins un composé choisi parmi : les agents desquamants, les agents anti-séborrhéiques, les agents matifiants, les agents anti-bactériens, les agents apaisants et leurs mélanges.
